# EUROPEAN PATENT SPECIFICATION

(11) **EP 3 450 353 B1**
(45) Date of publication and mention of the grant of the patent: **01.06.2022**
(21) Application number: 16900316.7
(22) Date of filing: 29.04.2016
(51) Int. Cl.: B65F 7/00, A61L 9/20, F25D 17/04, A61L 11/00

(54) **FREEZER FOR BIOSANITARY AND CYTOTOXIC WASTE**
GEFRIEREINRICHTUNG FÜR BIOSANITÄRE UND ZYTOTOXISCHE ABFÄLLEN
CONGÉLATEUR POUR DÉCHETS BIOSANITAIRES ET CYTOTOXIQUES

(43) Date of publication of application: 06.03.2019
(73) Proprietor: Bioseguridad Sanitaria Por Frio, S. L., 28046 Madrid (ES)
(72) Inventor: TORO CASASNOVAS, Eduardo, 28046 Madrid (ES)
(74) Representative: Capitán García, Maria Nuria
(86) International application number: PCT/ES2016/070330
(87) International publication number: WO 2017/186977

(56) References cited:
- CN-A- 105 292 856
- CN-A- 105 465 898
- CN-U- 203 473 687
- ES-U- 1 062 531
- FR-A1- 2 724 915
- KR-U- 20120 005 583
- US-A- 3 041 852
- US-A- 4 592 192
- US-A- 5 337 581
- US-A- 5 908 601

## Description

### FIELD OF THE INVENTION

The object of the present invention is a freezer for biosanitary and cytotoxic waste of the type used in hospitals, or for the transportation of such waste to incineration plants, using refrigeration means that are able to delay the degradation of such waste; incorporating as a novelty, an air purifier by titanium dioxide photocatalysis to cancel out the emissions of particulate contaminants produced by the waste contained in its interior.

### BACKGROUND OF THE INVENTION

Different designs of refrigerators for biosanitary and cytotoxic waste are known. For example, the one shown in Spanish utility model ES 1062531 U, published on 1 July 2006, which comprises a front door that allows insertion and removal of a container where the waste is deposited, an upper door to pour the waste into the container. The refrigerator has respective coils arranged on the side walls and rear wall, as well as a thermostat that regulates the temperature inside the refrigerator.

It is also known another refrigerated waste storage container, for example, the container shown in the patent document US5337581, which has a refrigeration unit for cooling or freezing the contents therein and an ultraviolet lamp for producing radiation to kill microorganisms in the waste. The container includes a sealable cover which incorporates the ultraviolet lamp and appropriate controls for cycling the lamp and the refrigeration unit to eliminate objectionable odors and germs.

Another example is the food waste container shown in the patent document KR20120005583, which comprising a container with a lid, and a garbage can places inside the container. The lower part of the garbage can comprises a plurality of holes which conform outlets of moisture and water contained in the food waste deposited in the garbage can. The water falls toward to a water tank arranged in the bottom of the container. A first sterilization lamp of the water contained in the water tank is arranged in its upper part, and a second sterilization lamp of food waste contained in the garbage can is arranged in the lid. The lamps radiate ultraviolet light to retard the deterioration of food waste and decay microorganisms, as well as reduce bad odors.

Another example is shown in the patent document CN105292856, which discloses an intelligent deodorant dustbin that comprising a shell, an inner barrel and a dustbin cover. The shell is made of metal and an electromagnet and an infrared induction switch are arranged on the dustbin cover. When a person approaches the dustbin, the infrared induction switch de-energizes the electromagnet, causing the release of an elastic piece which joints the dustbin cover to the shell, and the opening of the dustbin cover takes place to deposit the waste in the inner barrel. According to the intelligent deodorant dustbin, and the opening and closing method thereof, foul smell in the dustbin can be effectively prevented from being diffused.

Another example is shown in the utility model document CN203473687, which discloses an ultraviolet-light sterilization dustbin that includes a dustbin body and a dustbin cover. The dustbin cover is installed at the upper end of the dustbin body. The dustbin body includes an outer barrel and a transparent inner barrel which is installed in the outer barrel. An annular ultraviolet light is installed between the outer barrel and the transparent inner barrel. A transformer is installed in the dustbin cover. The dustbin cover and the outer barrel comprise two connection means that can be plugged together to connect the transformer with the ultraviolet lamp. When the dustbin cover is opened, the connection means are disconnected and the ultraviolet lamp is turned off; and the lamp is turned on when the dustbin cover is closed again. The utility model provides the ultraviolet-light sterilization dustbin which is improved in ultraviolet sterilization effect and great in practicality.

Another example is shown in the patent document US3041852, which discloses a refrigerated garbage storage device for domestic use, comprising an open topped cylindrical chamber with thermal insulation, which has a heat insulated closure connected to the cylindrical chamber by means of a hinge. Inside the device, a storage chamber of a typical garbage receptacle is formed, wherein the receptacle may be removably located. The cooling system of the device comprises a closed loop of flexible tubing having a heat absorbing portion encircling the inner metallic casing that conforms the storage chamber.

These known refrigerators have the disadvantage that, when opening either the upper door or the front door, both chemical and biological particulate contaminants are emitted to the outside environment together with the air stream coming from the interior of the refrigerator.

For this reason, a freezer for biosanitary and cytotoxic waste needs to be designed, which is able to simply and economically solve the disadvantage of the aforementioned prior art.

### DESCRIPTION OF THE INVENTION

The present invention is established and characterised in the independent claims, while the dependent claims describe additional features thereof.

The object of the invention is a freezer for biosanitary and cytotoxic waste. The technical problem to solve is how to prevent the emission of particulate contaminants to the outside environment, when opening a door of the freezer.

An advantage of the invention is that it manages to effectively provide a solution to the technical problem addressed, as the arrangement of the air purifier at the outlet of the freezer door, enables treatment of the stream of air coming from the inner space of the freezer when any of its doors has been opened, thus avoiding the emission of both chemical and biological particulate contaminants to the outside environment.

### BRIEF DESCRIPTION OF THE FIGURES

This specification is complemented with a figure that illustrates the preferred example of the invention, in no way intended to limit the scope thereof.

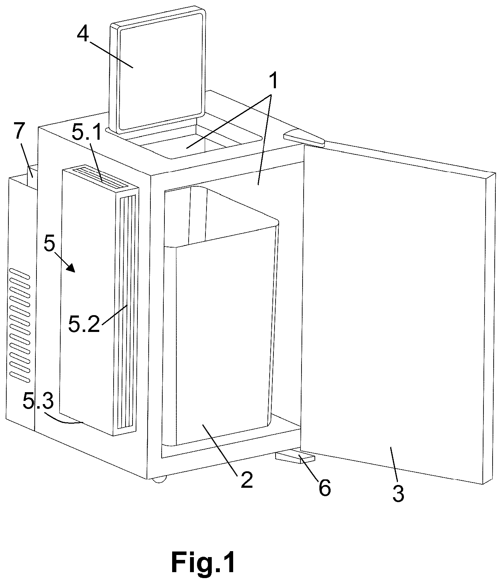

Figure 1 shows a perspective view of the freezer for biosanitary and cytotoxic waste.

### DETAILED DESCRIPTION OF THE PREFERRED EMBODIMENT OF THE INVENTION

This invention is a freezer for biosanitary and cytotoxic waste.

As shown in figure 1, the freezer comprises:
- an inner space (1), which houses a waste container (2),
- a front door (3), through which the waste container (2) in the inner space (1) is inserted/removed,
- an upper door (4), through which the waste is poured into the inner container (2),
- cooling means (7) and temperature control means (not shown in the figure) of the inner space (1).

For example, the cooling means (7) may comprise respective coils (not shown in the figure) arranged on the side walls and rear wall of the freezer; and, the temperature control means may be a thermostat (not shown in the figure) that regulates the temperature in the inner space (1) of the freezer between 0°C and -25°C.

Likewise, the freezer comprises an air purifier (5) that treats an air stream coming from the inner space (1). The air purifier (5) may be arranged on the outside of the freezer, for example, on one of its sides.

Preferably, the air purifiers (5) comprises an upper air inlet (5.1), oriented towards the upper door (4); similarly, it may comprise a front air inlet (5.2), oriented towards the front door (3) of the freezer.

Likewise, it is preferred that the air purifier (5) is put into operation when opening the upper door (4) or the front door (3). Being preferred that the upper door (4) or the front door (3) open by operating an accessible pedal (6), either at the front of the freezer or at one of its sides.

The air purifier (5) may comprise impeller means, for example, a fan (not shown in figure), that drag the air stream coming from the inner space (1) towards the air purifier (5), when the upper door (4) or front door (3) of the freezer is opened. At the same time, such impeller means transfer the air stream with particulate contaminants through the interior of the air purifier (5), between the upper air inlets (5.1) or front air inlets (5.2), and a purified air outlet (5.3).

On the other hand, it is preferred that the air stream coming from the inner space (1) is treated in the air purifier (5) by photocatalysis, for example, using titanium dioxide (TiO₂) as photo-catalyst. For this purpose, the air purifier (5) may comprise in its interior a matrix of titanium dioxide (TiO₂) (not shown in the figure) irradiated by ultraviolet (UV) lamps (not shown in the figure). The air stream transferred between the inlets (5.1, 5.2) and the outlet (5.3) of the air purifier (5) flows through the irradiated matrix.

In passing through the air purifier (5), the air stream with particulate contaminants, coming from the inner space (1) of the freezer, is converted into purified air at the outlet of the air purifier (5), cancelling out the emission to the outside environment of both chemical and biological particulate contaminants, which come from the biosanitary and cytotoxic waste in segregation phase contained in the container (2) of the inner space (1) of the freezer.

The cleaning of the air stream occurs when the accompanying particulate contaminants "oxidise" by contact with the hydroxyl radicals and superoxide ions formed when the titanium dioxide (TiO₂) absorbs the photons coming from the ultraviolet (UV) lamps, until managing to completely reduce these particulate contaminants to carbon dioxide (CO₂) and water vapour; obtaining as a result purified air suitable to be emitted to the outside environment, without risk of contamination thereof.

## Claims

1. Freezer for biosanitary and cytotoxic waste, comprising:
- an inner space (1), which houses a waste container (2),
- a front door (3), through which the waste container (2) in the inner space (1) is inserted/removed,
- an upper door (4), through which the waste is poured into the inner container (2),
- cooling means (7),
- inner space (1) temperature control means, and
- an air purifier (5) that treats an air stream coming from the inner space (1).
**characterised in that** the air purifier (5) is arranged on the outside of the freezer compartment comprising an upper air inlet (5.1) and a front air inlet (5.2), wherein, the upper air inlet (5.1) is oriented towards the upper door (4) and the front air inlet (5.2) is oriented towards the front door (3), wherein the air purifier (5) comprises an impeller means to drag the air stream coming from the inner space (1) towards the air purifier (5), when the upper door (4) or front door (3) of the freezer is opened.

2. Freezer according to claim 1, wherein the air purifier (5) is arranged on an outside surface of one of the freezer lateral sides.

3. Freezer according to claim 1, wherein the air purifier (5) is adapted to be put into operation when opening the upper door (4) or the front door (3).

4. Freezer according to claim 1, wherein the upper door (4) or the front door (3) open by operating a pedal (6) .

5. Freezer according to claim 1, wherein the air purifier (5) comprises photocatalyst means adapted to treat air stream coming from the inner space (1) by photocatalysis.

6. Freezer according to claim 5, wherein the photo-catalyst means are titanium dioxide (TiO₂).

7. Freezer according to claim 6, wherein the air purifier (5) comprises a matrix of titanium dioxide (TiO₂) irradiated by ultraviolet (UV) lamps.

8. Freezer according to claim 1, wherein the air stream impeller means comprise a fan.

## Patentansprüche

1. Gefriertruhe für biosanitäre und zytotoxische Abfälle, umfassend:
- einen Innenraum (1), der einen Abfallbehälter (2) aufnimmt,
- eine vordere Tür (3), durch die der Abfallbehälter (2) in den Innenraum (1) eingesetzt/daraus entnommen wird,
- eine obere Tür (4), durch die der Abfall in den Innenbehälter (2) geworfen wird,
- Kühlmittel (7),
- Mittel zur Temperaturregelung im Innenraum (1) und
- einen Luftreiniger (5), der einen aus dem Innenraum (1) kommenden Luftstrom behandelt,
**dadurch gekennzeichnet, dass** der Luftreiniger (5) an der Außenseite des Gefrierfachs angeordnet ist und einen oberen Lufteinlass (5.1) und einen vorderen Lufteinlass (5.2) umfasst, wobei der obere Lufteinlass (5.1) auf die obere Tür (4) ausgerichtet ist und der vordere Lufteinlass (5.2) auf die vordere Tür (3) ausgerichtet ist, wobei der Luftreiniger (5) ein Flügelradmittel umfasst, um den aus dem Innenraum (1) kommenden Luftstrom in den Luftreiniger (5) zu saugen, wenn die obere Tür (4) oder die vordere Tür (3) der Gefriertruhe geöffnet wird.

2. Gefriertruhe nach Anspruch 1, wobei der Luftreiniger (5) an einer Außenfläche einer der Seiten der Gefriertruhe angeordnet ist.

3. Gefriertruhe nach Anspruch 1, wobei der Luftreiniger (5) dafür ausgelegt ist, beim Öffnen der oberen Tür (4) oder der vorderen Tür (3) in Betrieb gesetzt zu werden.

4. Gefriertruhe nach Anspruch 1, wobei die obere Tür (4) oder die vordere Tür (3) durch Betätigen eines Pedals (6) geöffnet wird.

5. Gefriertruhe nach Anspruch 1, wobei der Luftreiniger (5) photokatalytische Mittel umfasst, die dafür ausgelegt sind, einen Luftstrom, der aus dem Innenraum (1) kommt, durch Photokatalyse zu behandeln.

6. Gefriertruhe nach Anspruch 5, wobei die photokatalytischen Mittel Titandioxid (TiO₂) sind.

7. Gefriertruhe nach Anspruch 6, wobei der Luftreiniger (5) eine Matrix aus Titandioxid (TiO₂) umfasst, die von Ultraviolett (UV)-Lampen bestrahlt wird.

8. Gefriertruhe nach Anspruch 1, wobei die Luftstrom-Flügelradmittel einen Ventilator umfassen.

## Revendications

1. Congélateur pour déchets biosanitaires et cytotoxiques comprenant :
- un espace interne (1) qui abrite un conteneur à déchets (2),
- une porte avant (3), à travers laquelle le conteneur à déchets (2) dans l'espace interne (1) est inséré/retiré,
- une porte supérieure (4), à travers laquelle les déchets sont déversés dans le conteneur interne (2),
- un moyen de refroidissement (7),
- un moyen de régulation de la température de l'espace interne (1) et
- un purificateur d'air (5) qui traite un flux d'air provenant de l'espace interne (1),
**caractérisé en ce que** le purificateur d'air (5) est agencé à l'extérieur du compartiment congélateur comprenant une entrée d'air supérieure (5.1) et une entrée d'air avant (5.2), dans lequel l'entrée d'air supérieure (5.1) est orientée vers la porte supérieure (4) et l'entrée d'air avant (5.2) est orientée vers la porte avant (3), dans lequel le purificateur d'air (5) comprend un moyen de turbine pour entraîner le flux d'air provenant de l'espace interne (1) vers le purificateur d'air (5) lorsque la porte supérieure (4) ou la porte avant (3) du congélateur est ouverte.

2. Congélateur selon la revendication 1, dans lequel le purificateur d'air (5) est agencé sur une surface extérieure de l'un des côtés latéraux du congélateur.

3. Congélateur selon la revendication 1, dans lequel le purificateur d'air (5) est adapté pour être mis en fonctionnement lors de l'ouverture de la porte supérieure (4) ou de la porte avant (3).

4. Congélateur selon la revendication 1, dans lequel la porte supérieure (4) ou la porte avant (3) s'ouvre par actionnement d'une pédale (6).

5. Congélateur selon la revendication 1, dans lequel le purificateur d'air (5) comporte un moyen photocatalyseur adapté pour traiter le flux d'air provenant de l'espace interne (1) par photocatalyse.

6. Congélateur selon la revendication 5, dans lequel le moyen photocatalyseur est du dioxyde de titane (TiO₂) .

7. Congélateur selon la revendication 6, dans lequel le purificateur d'air (5) comprend une matrice de dioxyde de titane (TiO₂) irradiée par des lampes ultraviolettes (UV).

8. Congélateur selon la revendication 1, dans lequel le moyen de turbine à flux d'air comprend un ventilateur.
